# EUROPEAN PATENT APPLICATION

(11) **EP 4 147 658 A1**
(43) Date of publication of application: **15.03.2023**
(21) Application number: 22188500.7
(22) Date of filing: 03.08.2022
(51) Int. Cl.: A61B 18/14, A61N 1/06, A61N 1/32, A61N 1/40, A61B 18/00

(54) **DOUBLE MONOPOLAR RF BODY CONTOURING**

(30) Priority: 13.09.2021 US 202163243219 P; 02.08.2022 US 202217878946
(71) Applicant: Intelis Instruments Ltd., 3850172 Hadera (IL)
(72) Inventor: VAYNBERG, Boris, 3090000 Zichron Yakov (IL)
(74) Representative: Durán-Corretjer, S.L.P.

(57) **Abstract**

Disclosed a device for body contouring treatment. The device (200) comprisies a layer of electrically conductive material (204), bounded by a frame (208) with cooling fluid conducting channels (212); and a ceramic material layer (216) with first side (220) configured to contact the conductive material layer (204) and a second side (224) configured to contact a treated skin surface (228), wherein the dimensions of the ceramic material layer (216) exceed the dimensions of the layer of electrically conductive material (204) by at least 5 mm in each direction.

## Description

### TECHNOLOGY FIELD

The present device and method relate to cosmetic tissue treatment by RF energy and, in particular, to body contouring by high-frequency RF energy.

### BACKGROUND

Radiofrequency (RF) is a popular and effective method of tightening skin and reducing wrinkles currently available treatment. Radiofrequency delivers heat energy to the upper and middle levels of the skin, stimulating new collagen growth.

One or more electrical current conducting electrodes applied to the skin and connected to an RF generator deliver the heat energy to the skin. Typically, the RF energy is applied to the skin in a pulse mode. The pulses could be short, for example, microseconds, or long, like tens of seconds.

Two types of RF skin treatment are in use. Monopolar RF skin treatment where the radiofrequency energy travels from an active electrode to a distant and large passive electrode. Bipolar RF skin treatment where the radiofrequency energy alternates between two electrodes situated at a short distance from one another. Both types of RF are used for skin rejuvenation and reduction of subcutaneous fat.

High-frequency RF tissue treatment uses low-current high-frequency alternating currents typically delivered via a glass electrode without direct electrical contact. Different skin conditions, such as fine lines and wrinkle removal, acne management, faded dark eye circles, etc., could be treated by high-frequency RF.

The operation of high-frequency machines requires high-frequency RF electrodes, which come as a clear glass electrodes in different shapes and sizes to treat different treatment areas of the body and face. The electrodes could be hollow and filled with Argon or Neon gas.

### DEFINITIONS

The term "body contouring" includes a wide range of tissue treatments and procedures intended to reshape, redefine, tighten, tone, and/or improve a person's overall appearance.

The present device utilizes a "double monopolar" approach where two electrodes of equal dimensions are positioned at a distance larger than dimension of the electrode from each other on the skin surface.

### SUMMARY

A device for body contouring treatment includes a layer of electrically conductive material, bounded by a frame with cooling fluid conducting channels. The device also includes a ceramic material layer, with a first side configured to contact the conductive material layer and a second side configured to contact a treated skin surface. The dimensions of the ceramic material layer exceed the dimensions of the layer of electrically conductive material by at least 5 mm in each direction. The first side and the second side of the ceramic layer may be planar. The ceramic material layer may thereby extend beyond the layer of electrically conductive material in the plane of the first side. The cooling fluid conducting channels may be one of a group of channels monolithic with the ceramic layer or attached to the ceramic layer. The device may also include at least one temperature sensor embedded in the ceramic material layer at the periphery of the electrically conductive material.

The frame with cooling fluid conducting channels may be made from an electrically non-conductive material having good thermal conductivity such as sapphire or a thermally conductive ceramic material, examples being silicon nitride, beryllium oxide or aluminium nitride. Sapphire has both a high thermal conductivity and high electrical resistance and is therefore advantageous for dissipating heat. The device may be adapted for use as an electrode for high-frequency RF skin contouring and with a connection to a source of high-frequency RF. The device could be used as an electrode for high-frequency RF skin contouring. The shape and size of the device could be adapted to the shape and size of the treated anatomical position.

Described is also a system for body contouring treatment. The system includes an RF frequency generator adapted to generate high-frequency RF energy with the frequency of 20-50 MHz and supply the high-frequency RF energy to at least one high-frequency RF electrode. The high-frequency RF electrode includes a conductive layer and a ceramic layer, with the dimensions of the ceramic layer exceeding the dimensions of the conductive layer by at least 5 mm in each direction. A ceramic cooling fluid conducting channels are arranged on the peripheral edges of the conductive layer.

For body contouring, the system typically includes two similar or identical high-frequency RF electrodes applied to the skin and located such on the skin that the distance between the two high-frequency RF electrodes exceeds the dimension of the high-frequency RF electrode. The system may operate in a double monopolar mode.

### LIST OF FIGURES AND THEIR SHORT DESCRIPTION

FIG. 1 is an example of a "hot spot" formed along the edges of an RF electrode;
FIG. 2 is an example of a present high-frequency RF electrode alleviating the "hot spot" around the electrode;
FIG. 3 is an example of the positioning of the present high-frequency RF electrode for body contouring; and
FIG. 4 is an example of a high-frequency body contouring system.

### DESCRIPTION

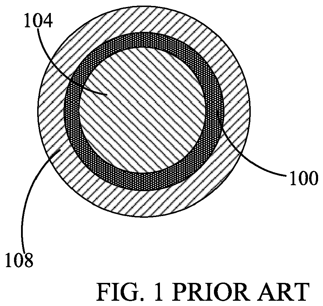

As noted above, the RF treatment utilizes electrically conductive electrodes to deliver RF power to the treated area of the skin. Electrically conductive electrodes lead to a specific electrical current distribution from the electrode to the tissue. The edges of the RF electrodes are much hotter than the middle of the electrode. It is customary to say that such temperature distribution has a "hot spot" 100 located at the edge of the RF electrode. FIG. 1 is an example of a "hot spot" formed along the edges of an RF electrode 104. The picture is an illustration of the thermal image of the tissue immediately after the RF treatment. The "hot spot" 100 limits the dwell time for RF energy application to the same skin location and presents an overheating danger to the upper layer of the skin. And therefore, the "hands-free" stationary usage of RF devices is impossible. Most RF devices are used "in motion" where an applicator with RF electrodes moves to average the heating across the skin.

The edge hot spot effect exists for any frequency and conductive and capacitive energy coupling to the tissue. For the low RF frequencies between 0.2-5 Mhz, the RF electrodes touch the skin directly and frequently use a coupling gel. For the higher RF frequencies from 5 to 50 MHz, an isolator layer is between the electrode and the tissue, and the coupling is capacitive.

The "hot spot" 100 is illustrated as a regular round area, although practically, it could have different widths varying along the electrode 104 perimeter. Reference numeral 108 shows the surrounding tissue schematically.

Some known stationary devices typically operate at a duty cycle of less than 50% "ON" time. Such devices use the "OFF" time to allow the temperature to decrease by dissipating the heat into tissue. The adequate amount of energy delivered to the skin is small, and the deeper skin layers are almost unaffected.

In existing energy-based skin treatment devices, the energy emitted by the heat source (laser, RF, or ultrasound) is absorbed in the upper layers of tissue. The upper skin layers absorb most of the energy. Therefore, the deeper layers are heated by conductivity, and the treatment usually takes a long time (0.5-1 hour). Cooling of the heated upper skin layers requires the use of a significant tissue cooling mechanism.

The present tissue treatment device utilizes high-frequency RF to heat the full thickness of a fat layer simultaneously and not by thermal conductivity from the upper layers. The use of the high-frequency RF provides a more homogeneous distribution of heat caused by RF in the tissue and supports an accurate control of the skin temperature. The high-frequency RF application to the skin supports simultaneous fat removal and skin tightening.

The "monopolar" RF application penetrates the tissue for 20-50 mm. The disadvantage of standard monopolar RF application is that the return current at low RF frequencies returns through a large return electrode. In high-frequency RF the return current returns through the air and does not cause additional clinical effects. The present device utilizes a "double monopolar" approach where two electrodes of equal dimensions are positioned at a distance from each other on the skin surface. The separation between the two high-frequency RF electrodes significantly exceeds the electrode's dimensions and the fat layer's thickness.

Preservation of epidermal layer integrity minimizes recovery and the risk of complications. The authors of the disclosure believe that monopolar radiofrequency capacitively coupled to the skin does not cause epidermal problems and is advantageous for treatments designed for body contouring applications.

The present document discloses a high-frequency RF electrode with peripheral cooling supporting the protection of the tissue from overheating. Additionally, the high-frequency RF electrode supports delivery and coupling to the tissue of the high-frequency RF energy in a non-contact way.

The present document also discloses using a high-frequency RF electrode in a "double monopolar" approach. The separation between the two high-frequency RF electrodes and their shape facilitates skin tightening and fat removal.

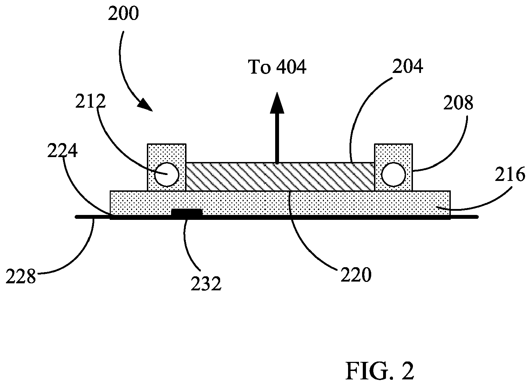

FIG. 2 is an example of a present high-frequency RF electrode alleviating the formation of the "hot spot" around the RF electrode. High-frequency RF electrode 200 includes a layer of electrically conductive material 204, bound by a frame 208 with cooling fluid conducting channels 212. In some examples, frame 208 with cooling fluid conducting channels 212 could be one of a group of monolithic frames with the ceramic layer 216 or attached to the ceramic layer 216. The ceramic material layer could be one of a group of materials such as alumina, barium titanate, and Sapphire. In other examples, frame 208 could be manufactured of an electrically insulating and heat conducting material.

It is not necessary to cool the whole area of the RF electrode because due to the current distribution in the RF electrode, the hot spot is only at the edges of the RF electrode. The layer of electrically conductive material 204, which could be a metal layer, is mounted on a ceramic material layer 216 with a first side 220 configured to contact conductive material 204 and a second side 224 configured to contact a treated skin surface 228. Both the first and the second sides of the ceramic material layer 216 are planar. The dimensions of the ceramic material layer 216 exceed the dimensions of the layer of electrically conductive material 204 by at least 5 mm in each direction, i.e. in each direction within the plane of the first side 220. The layer of electrically conductive material 204 could have a surface of 10 to 30 mm². One or more temperature sensors 232 could be attached to the ceramic layer 216. Temperature sensors could be mounted along the perimeter of the electrically conductive material 204, which usually be metal.

FIG.2 illustrates device 200 having a circular shape. However, device 200 could be of different shapes and sizes suitable for treatments in different areas of a body or in more general terms, adapted to the anatomical position of the body. To avoid charge concentrations, the electrodes are typically round or have rounded corners and may for example have a rectangular shape with rounded corners or an elliptical shape.

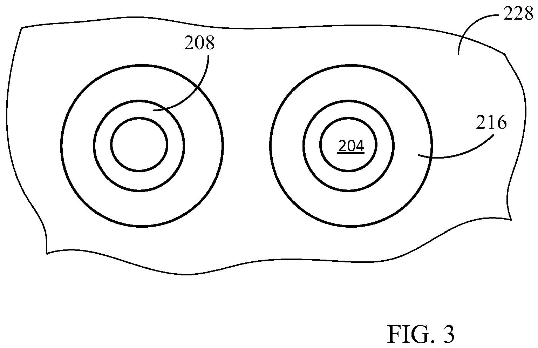

The high-frequency RF electrode 200 is positioned on the skin's surface for the body contouring treatment. FIG. 3 is an example of positioning on the skin surface of the present high-frequency RF electrodes for body contouring. The distance between the electrodes 200-1 and 200-2 is comparable to the dimensions of the electrodes. With such a high-frequency RF electrodes arrangement, tissue heating dominates the treatment and allows targeting the different tissue layers, achieving the clinical results in fat removal and skin tightening.

The distance between the two electrodes 200-1 and 200-2 may be larger than the dimensions of the electrodes; for example, it could be 20 to 50 mm and usually would be 30 mm.

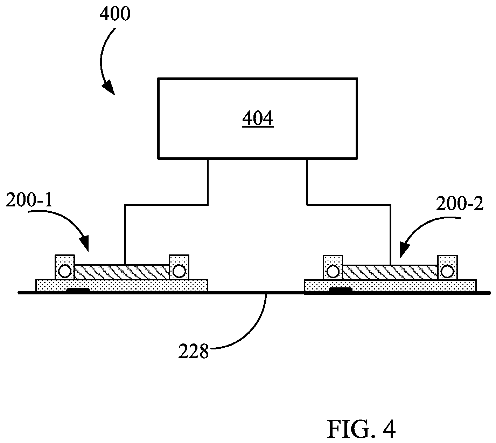

FIG. 4 is an example of a high-frequency body contouring system. System 400 includes a high-frequency RF generator 404; at least one RF electrode 200-1 comprising a conductive layer and a ceramic layer with the dimensions of the ceramic layer exceeding the dimensions of the conductive layer on at least 5 mm in each direction; and a ceramic cooling fluid conducting channels arranged on the peripheral edges of the conductive layer.

The high RF frequency generator 404 generates RF energy with a frequency of 20-50 MHz. System 400 includes at least one additional electrode 200-2 similar to at least one RF electrode 200-1 and located from the one electrode on a distance exceeding the dimension of the electrode. System 400, operates in a double monopolar mode.

The described system is suitable for body contouring treatments, and the electrode could be applied to other RF skin treatments, where the capacitive coupling to the skin is advantageous.

Several examples have been described. Nevertheless, it will be understood that various modifications may be made without departing from the disclosed method, device's spirit, scope, and method of use. Accordingly, other examples are within the scope of the following claims.

## Claims

1. A device (200) for body contouring treatment, comprising:
a layer of electrically conductive material (204), bounded by a frame (208) with cooling fluid conducting channels (212); and
a ceramic material layer (216) with a first side (220) configured to contact the conductive material layer (204) and a second side (224) configured to contact a treated skin surface (228),
wherein dimensions of the ceramic material layer (216) exceed the dimensions of the layer of electrically conductive material (204) by at least 5 mm in each direction.

2. The device (200) of claim 1, wherein the electrically insulating cooling fluid conducting channels (212) are one of a group of channels monolithic with the ceramic material layer (216) or attached to the ceramic layer (216).

3. The device (200) of claim 1 or claim 2, wherein at least one temperature sensor (232) is embedded in the ceramic material layer (216) at a periphery of the electrically conductive material layer (204).

4. The device (200) of any one of claims 1 to 3, where the frame (208) with cooling fluid conducting channels (212) is made from an electrically non-conductive material having good thermal conductivity such as sapphire or a ceramic material.

5. The device (200) of any one of claims 1 to 4, wherein the shape and size of the device (200) are adapted to the shape and size of a treated anatomical position.

6. The device (200) of any one of claims 1 to 5 further comprising a connection to a source of high-frequency RF generator (404) configured to supply high-frequency RF to the device (200).

7. The device (200) of any one of claims 1 to 6, wherein the first side and the second side (224) of the ceramic material layer (216) are planar.

8. The device (200) of any one of claims 1 to 7, wherein the ceramic material layer (216) is one of a group of materials such as alumina, barium titanate, and sapphire, and/or wherein the layer of electrically conductive material (204) is a metal layer.

9. The device (200) of any one of claims 1 to 8, wherein the device (200) is an electrode for high-frequency RF skin contouring.

10. A system (400) for body contouring treatment, comprising:
an RF frequency generator (404) adapted to generate high-frequency RF;
at least one high-frequency RF electrode (200-1, 200-2) comprising a conductive material layer (204) and a ceramic material layer (216) with dimensions of the ceramic material layer (216) exceeding the dimensions of the conductive layer (204) by at least 5 mm in each direction; and
cooling fluid conducting channels (212) arranged on peripheral edges of the conductive material layer (204).

11. The system (400) according to claim 10, wherein the high-frequency RF generator (404) generates RF energy with the frequency of 20-50 MHz.

12. The system (400) according to claim 10 or claim 11, wherein the system (400) includes at least one additional high-frequency RF electrode (200-1, 200-2) similar to the at least one RF electrode (200-1, 200-2) and located from the one RF electrode (200-1, 200-2) at a distance exceeding the dimension of the RF electrode (200-1, 200-2).

13. The system (400) according to any one of claims 11 to 12, wherein the system (400) operates in a double monopolar mode and/or wherein one of the high-frequency RF electrodes (200-1, 200-2) is connected to one pole of the high-frequency RF generator (404) and another high-frequency RF electrode (200-1, 200-2) is connected to another pole of the high-frequency RF generator (404).

14. The system (400) according to any one of claims 10 to 13, wherein the high frequency electrodes (200-1, 200-2) are applied to tissue in a way where the separation between the electrodes (200-1, 200-2) is larger than the dimension of the electrodes (200-1, 200-2).

15. A method of high-frequency skin contouring treatment, comprising:
applying to skin (228) at least one high-frequency RF electrode (200-1, 200-2) comprising a conductive material layer (204) and a ceramic material layer (216) with dimensions of the ceramic material layer (216) exceeding the dimensions of the conductive material layer (204) by at least 5 mm in each direction;
capacitively coupling the high-frequency RF electrode (200) to the skin (228); and
delivering to the skin (228) a high-frequency RF energy to cause a skin contouring treatment.
